# EUROPÄISCHE PATENTSCHRIFT

(11) **EP 2 178 497 B1**
(45) Veröffentlichungstag und Bekanntmachung des Hinweises auf die Patenterteilung: **22.11.2017**
(21) Anmeldenummer: 08803034.1
(22) Anmeldetag: 14.08.2008
(51) Int. Cl.: A61Q 5/02, A61Q 5/12, A61K 8/73, A61K 8/898

(54) **KOSMETISCHE MITTEL MIT CHITOSAN UND SILIKONELASTOMEREN**
COSMETIC COMPOSITIONS WITH CHITOSAN AND SILICONE ELASTOMERS
PRODUITS COSMÉTIQUES À BASE DE CHITOSANE ET DE SILICONES ÉLASTOMÈRES

(30) Priorität: 21.08.2007 DE 102007039519
(43) Veröffentlichungstag der Anmeldung: 28.04.2010
(73) Patentinhaber: Henkel AG & Co. KGaA, 40589 Düsseldorf (DE)
(72) Erfinder: FLODROP VAN, Helga, 21075 Hamburg (DE); HENTRICH, Dirk, 22457 Hamburg (DE)
(86) Internationale Anmeldenummer: PCT/EP2008/060683
(87) Internationale Veröffentlichungsnummer: WO 2009/024524

(56) Entgegenhaltungen:
- EP-A- 0 524 612
- EP-A- 0 760 235
- EP-A- 1 584 323
- WO-A-99/22701
- DE-A1- 10 221 449
- DE-A1- 19 850 734
- DE-C1- 4 343 378
- JP-A- 2007 008 867
- GOTTSCHALCK T E ET AL: "INTERNATIONAL COSMETIC INGREDIENT DICTIONARY AND HANDBOOK, passage" INTERNATIONAL COSMETIC INGREDIENT DICTIONARY AND HANDBOOK, XX, XX, 1. Januar 2006 (2006-01-01), Seiten 2148-2152, XP002470374
- "Dow Corning launches new silicone for hair care" INTERNET CITATION, [Online] 11. Mai 2005 (2005-05-11), Seite 1, XP007910350 Gefunden im Internet: URL:http://www.cosmeticsdesign.com/content /view/print/146660> [gefunden am 2009-10-28]
- DATABASE GNPD [Online] MINTEL; 1 April 2007 (2007-04-01), Samy: "Fat Hair "0" Calories Thickening Hair Serum", Database accession no. 692019
- SpecialChem: "Paper Discusses Performance of Dow Corning® 5-7070 Silicone Amino Elastomer Emulsion", , 7 June 2006 (2006-06-07), Retrieved from the Internet: URL:http://cosmetics.specialchem.com/news/ product-news/paper-discusses-performance-o f-dow-corning-5-7070-silicone-amino-elasto mer-emulsion [retrieved on 2015-03-12]

## Beschreibung

Die vorliegende Erfindung betrifft Haarbehandlungsmittel und deren Verwendung. Insbesondere betrifft die vorliegende Erfindung Haarreinigungs- und -konditioniermittel zur Verbesserung des Haarzustandes.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst lang anhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein oder die Haare vor erhöhtem Spliß geschützt sein.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splißrate verringert.

Weiterhin wurden in jüngster Zeit so genannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate wirken im allgemeinen bevorzugt an der Haaroberfläche. So sind Wirkstoffe bekannt, welche dem Haar Glanz, Halt, Fülle, bessere Naß- oder Trockenkämmbarkeiten verleihen oder dem Spliß vorbeugen. Genauso bedeutend wie das äußere Erscheinungsbild der Haare ist jedoch der innere strukturelle Zusammenhalt der Haarfasern, der insbesondere bei oxidativen und reduktiven Prozessen wie Färbung und Dauerwellen stark beeinflußt werden kann.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften und guter biologischer Abbaubarkeit. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen. Insbesondere Wirkstoffe, die dem Haar Fülle und Volumen geben, zeichnen sich zu einem großen Teil dadurch aus, daß die Effekte mit der Zeit nachlassen und nicht den gesamten Zeitraum zwischen zwei Haarbehandlungen abdecken. Dieser Verlust von Fülle und Volumen - beispielsweise während eines Arbeitstages - wird von vielen Verbrauchern als unerwünscht empfunden. Die EP-A-0760235 offenbart Polymerkombinationen für Haarfestiger bestehend aus Chitosan-PCA und weiteren haarfestigenden Polymeren.

Die DE-A1-19850734 offenbart für Ethanol-haltige Haarfestigungsmittel geeignete Chitosan-Salze, darunter Chitosan-PCA.

Die WO-A1-99/22701 offenbart Haarstyling/-konditionierungsmittel, die anionische und zwitterionische Polymere in Kombination enthalten. Diese Mittel liefern einen langanhaltenden Frisurenhalt und eine verbesserte Nasskämmbarkeit. Die Beispiele 5 und 6 der D1 betreffen Zusammensetzungen, die neben 0,25 Gew.-% Carboxybutylchitosan zusätzlich einen Silikonrohstoff der Kennzeichnung DC 2-5791 enthalten.

In der EP-A2-524 612 werden Haarkosmetika offenbart, die Poly(N-Acylalkylenimin)-modifizierte Silikone in Kombination mit filmbildenden Polymeren enthalten. Beispiel 3 beschreibt eine Zusammensetzung, die 5,0 Gew.-% einer Chitosanlösung und 1,0 Gew.-% eines Poly(N-Acylalkylenimin)-modifizierten Silikons des Synthesebeispiels 1 enthält

Die DE-C1-43 43 378 betrifft konditionierende Haarpflegemittel, enthaltend eine Kombination aus
a) 0,1 bis 5,0 Gew.-% mindestens einer quaternären Ammoniumverbindung mit ein oder 2 (C₁ bis C₆)-Acylresten,
b) 0,05 bis 5,0 Gew.-% mindestens eines kationischen Polymeren und
c) 0,01 bis 5,0 Gew.-% mindestens eines speziellen wasserlöslichen Chitosans.

Die DE-A1-102 21 449 betrifft ein Aerosolschaum-/Pumpschaumprodukt für die Haarbehandlung, umfassend eine Zusammensetzung mit einem Gehalt an mindestens einem kationischen Cellulosederivat, mindestens einem Chitosan in speziellem Mengenverhältnis, mindestens einer Säure zum Neutralisieren des Chitosans und einem Lösemittelsystem.

Die EP-A1-1 584 323 betrifft die Verwendung spezieller Chitosanderivate (N-Hydroxyalkyl-O-benzylchitosan) zur Haarbehandlung und entsprechende Kosmetika mit diesen Chitosanderivaten in Kombination mit mindestens einem weiteren Haarbehandlungswirkstoff.

In Beispiel 6 der JP-A-2007 008867 werden Zusammensetzungen offenbart, die neben Chitosan 2 Gew.% eines Amino-modifizierten Silikons enthalten.

Aufgabe der vorliegenden Erfindung war die Bereitstellung von Haarbehandlungsmitteln, welche dem mit ihnen behandelten Haar eine verbesserte Fülle und ein gesteigertes Volumen geben. Insbesondere sollte der Halt der Frisur über einen längeren Zeitraum (Langzeithalt) verbessert werden, so daß die Fülle und das Volumen auch über einen längeren Zeitraum anhalten.

Es wurde nun gefunden, daß sich bestimmte Wirkstoffkombinationen aus Silikon und Chitosan vorteilhaft in Haarbehandlungsmittel einarbeiten lassen und neben vorteilhaften Produkt- und Produktionseigenschaften auch Fülle, Volumen und Langzeithalt deutlich verbessern.

Gegenstand der vorliegenden Erfindung sind in einer ersten Ausführungsform Haarbehandlungsmittel, enthaltend - bezogen auf ihr Gewicht -
a) 0,05 bis 5 Gew.-% mindestens eines Chitosans und/oder Chitosanderivaates und
b) 0,05 bis 5 Gew.-% mindestens eines elastomeren Silans oder Siloxans mit quaternären Ammoniumgruppen im Molekül in Form einer Öl-in-Wasser (O/W-) oder Wasser-in-ÖI (W/O-) Emulsion oder Microemulsion, bei der das Silan oder Siloxan die Ölphase bildet,
   wobei das bzw. die elastomeren Silan(e) oder Siloxan(e) mit quaternären Ammoniumgruppen im Molekül erhalten wurden durch Reaktion von
   (i) einer organischen quaternären Ammoniumverbindung mit Epoxidgruppen oder Halohydringruppen, wobei die organische quaternäre Ammoniumverbindung mit Epoxidgruppen ausgewählt ist aus Glycidyl-trimethylammoniumchlorid und/oder Glycidyl-trimethylammoniumbromid und wobei die die organische quaternäre Ammoniumverbindung mit Halohydringruppen ausgewählt ist aus
      - (3-Chlor-2-hydroxypropyl)trimethylammoniumchlorid,
      - (3-Chlor-2-hydroxypropyl)dimethyldodecylammoniumchlorid,
      - (3-Chlor-2-hydroxypropyl)dimethyloctadecylammoniumchlorid,
      - (3-Chlor-2-hydroxypropyl)trimethylammoniumbromid,
      - (3-Chlor-2-hydroxypropyl)dimethyldodecylammoniumbromid,
      - (3-Chlor-2-hydroxypropyl)-dimethyloctadecylammoniumbromid mit
   (ii) einem Silan oder Siloxan mit Aminogruppen, in Gegenwart von
   (iii) einem Verzweigungs-Agens, wobei das Verzweigungs-Agens ausgewählt ist aus Butandioldiglycidylether und/oder Polypropylenglycoldiglycidylether, und
   (iv) einem Tensid, wobei das Tensid ausgewählt ist aus ethoxylierten Alkoholen der Formel

      CnH2ₙ₊₁(OCH₂CH₂)ₓOH

      in der n für Zahlen zwischen 8 und 20, und x für Zahlen von 8 bis 18 stehen, dispergiert in
   (v) einer wäßrigen polaren Phase,
   wobei das Mittel
   0,1 bis 4,5 Gew.-% eines Neutralisationsproduktes von Chitosan mit Pyrrolidoncarbonsäure enthält.

Haarbehandlungsmittel im Sinne der vorliegenden Erfindung sind beispielsweise Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen. Bevorzugte erfindungsgemäße Mittel sind Shampoos, Konditioniermittel oder Haar-Tonics.

Die erfindungsgemäßen Haarbehandlungsmittel enthalten - bezogen auf ihr Gewicht- 0,05 bis 5 Gew.-% mindestens eines Chitosans und/oder Chitosanderivates.

Chitosane stellen Biopolymere dar und werden zur Gruppe der Hydrokolloide gezählt. Chemisch betrachtet, handelt es sich um partiell deacetylierte Chitine unterschiedlichen Molekulargewichtes. Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineral-säuren demineralisiert und schließlich durch Zugabe von starken Basen deacetyliert, wobei die Molekulargewichte über ein breites Spektrum verteilt sein können. Vorzugsweise werden solche Typen eingesetzt, die ein durchschnittliches Molekulargewicht von 800.000 bis 1.200.000 Dalton, eine Viskosität nach Brook-field (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen.

Die erfindungsgemäßen Haarbehandlungsmittel enthalten 0,1 bis 4,5 Gew.-% eines Neutralisationsproduktes von Chitosan mit Pyrrolidoncarbonsäure. Erfindungsgemäß bevorzugte Haarbehandlungsmittel sind dadurch gekennzeichnet, daß sie - bezogen auf ihr Gewicht - 0,25 bis 4 Gew.-%, besonders bevorzugt 0,5 bis 3,5 Gew.-%, weiter bevorzugt 0,75 bis 3 Gew.-% und insbesondere 1 bis 2 Gew.-% eines Neutralisationsproduktes von Chitosan mit Pyrrolidoncarbonsäure enthalten.

Die erfindungsgemäßen Haarbehandlungsmittel enthalten zusätzlich - bezogen auf ihr Gewicht - 0,05 bis 5 Gew.-% mindestens eines elastomeren Silans oder Siloxans mit quaternären Ammoniumgruppen im Molekül in Form einer ÖI-in-Wasser (O/W-) oder Wasser-in-ÖI (W/O-) Emulsion oder Microemulsion, bei der das Silan oder Siloxan die Ölphase bildet,
wobei das bzw. die elastomeren Silan(e) oder Siloxan(e) mit quaternären Ammoniumgruppen im Molekül erhalten wurden durch Reaktion von
(i) einer organischen quaternären Ammoniumverbindung mit Epoxidgruppen oder Halohydringruppen, wobei die organische quaternäre Ammoniumverbindung mit Epoxidgruppen ausgewählt ist aus Glycidyl-trimethylammoniumchlorid und/oder Glycidyl-trimethylammoniumbromid und wobei die die organische quaternäre Ammoniumverbindung mit Halohydringruppen ausgewählt ist aus
   - (3-Chlor-2-hydroxypropyl)trimethylammoniumchlorid,
   - (3-Chlor-2-hydroxypropyl)dimethyldodecylammoniumchlorid,
   - (3-Chlor-2-hydroxypropyl)dimethyloctadecylammoniumchlorid,
   - (3-Chlor-2-hydroxypropyl)trimethylammoniumbromid,
   - (3-Chlor-2-hydroxypropyl)dimethyldodecylammoniumbromid,
   - (3-Chlor-2-hydroxypropyl)-dimethyloctadecylammoniumbromid mit
(ii) einem Silan oder Siloxan mit Aminogruppen, in Gegenwart von
(iii) einem Verzweigungs-Agens, wobei das Verzweigungs-Agens ausgewählt ist aus Butandioldiglycidylether und/oder Polypropylenglycoldiglycidylether, und
(iv) einem Tensid, wobei das Tensid ausgewählt ist aus ethoxylierten Alkoholen der Formel

   CnH₂ₙ₊₁(OCH₂CH₂)ₓOH

   in der n für Zahlen zwischen 8 und 20, und x für Zahlen von 8 bis 18 stehen, dispergiert in
(v) einer wäßrigen polaren Phase,

Elastomere Silane oder Siloxane sind formfest, aber elastisch verformbar und weisen einen Glasübergangspunkt auf, der sich unterhalb der Raumtemperatur befindet.

Die erfindungsgemäß einsetzbaren Silikonelastomere sind breit kommerziell verfügbar, wobei sie in Form von Emulsionen in die erfindungsgemäßen Mittel inkorporiert werden.

Es hat sich gezeigt, daß ein besonders guter Langzeithalt bei hoher Fülle und hohem Volumen mit besonderen Silikonelastomeren erzielt werden kann. Diese speziellen Silikonelastomere sind im Rahmen der vorliegenden Erfindung ganz besonders bevorzugt, da sie in Kombination mit dem Chitosan(derivat) außergewöhnliche gute Effekte bereits bei niedrigen Einsatzkonzentrationen bewirken.

Erfindungsgemäß besonders bevorzugte Haarbehandlungsmittel sind daher dadurch gekennzeichnet, daß das bzw. die elastomeren Silan(e) oder Siloxan(e) mit quaternären Ammoniumgruppen im Molekül erhalten wurden durch Reaktion von
(i) einer organischen quaternären Ammoniumverbindung mit Epoxidgruppen oder Halohydringruppen, wobei die organische quaternäre Ammoniumverbindung mit Epoxidgruppen ausgewählt ist aus Glycidyl-trimethylammoniumchlorid und/oder Glycidyl-trimethylammoniumbromid und wobei die organische quaternäre Ammoniumverbindung mit Halohydringruppen ausgewählt ist aus
   - (3-Chlor-2-hydroxypropyl)trimethylammoniumchlorid,
   - (3-Chlor-2-hydroxypropyl)dimethyldodecylammoniumchlorid,
   - (3-Chlor-2-hydroxypropyl)dimethyloctadecylammoniumchlorid,
   - (3-Chlor-2-hydroxypropyl)trimethylammoniumbromid,
   - (3-Chlor-2-hydroxypropyl)dimethyldodecylammoniumbromid
   - (3-Chlor-2-hydroxypropyl)-dimethyloctadecylammoniumbromid mit
(ii) einem Silan oder Siloxan mit Aminogruppen, in Gegenwart von
(iii) einem Verzweigungs-Agens, wobei das Verzweigungs-Agens ausgewählt ist aus Butandioldiglycidy<lether und/oder Propylenglycoldiglycidylether, und
(iv) einem Tensid, wobei das Tensid ausgewählt ist aus ethoxylierten Alkoholen der Formel

   CnH₂ₙ₊₁(OCH₂CH₂)ₓOH

   In der n für Zahlen zwischen 8 und 20, und x für Zahlen von 8 bis 18 stehen, dispergiert in
(v) einer wäßrigen polaren Phase.

Bevorzugte Siloxane mit Aminogruppen lassen sich durch die Formel (SIL-I) beschreiben 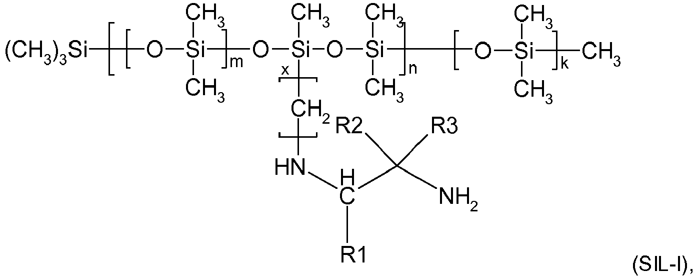 in der die Indices k, m und n so gewählt sind, daß das Siloxan 100 bis 5000 Si-Atome umfaßt. Die Indices k, m und n stehen dabei vorzugsweise in einem solchen Verhältnis zueinander, daß die -(CH₂)ₓ-NH-CH(R¹)-C(R²)(R³)-NH₂ Gruppen 1 bis 5 Mol.-%, vorzugsweise 1,5 bis 4 Mol.-%, weiter bevorzugt 2 bis 3 Mol.-% und insbesondere 2 bis 2,5 Mol.-% ausmachen.

X steht für eine Zahl 1, 2 oder 3, R¹, R² und R³ stehen unabhängig voneinander für H oder -CH₃.

Bevorzugte Gruppen -(CH₂)ₓ-NH-CH(R¹)-C(R²)(R³)-NH₂ sind Aminomethyl-aminoethylgruppen, Aminomethyl-aminpropylgruppen, Aminomethyl-aminoisopropylgruppen, Aminomethylaminobutylgruppen, Aminomethyl-aminoisobutylgruppen, Aminoethyl-aminoethylgruppen, Aminoethyl-aminpropylgruppen, Aminoethyl-aminoisopropylgruppen, Aminoethyl-aminobutylgruppen, Aminoethyl-aminoisobutylgruppen, Aminpropyl-aminoethylgruppen, Aminopropyl-aminpropylgruppen, Aminopropyl-aminoisopropylgruppen, Aminopropyl-aminobutylgruppen, Aminopropylam inoisobutylgruppen.

Besonders bevorzugte Siloxane mit Aminogruppen lassen sich durch die Formeln (SIL-Ia) bis (SIL-If) beschreiben:

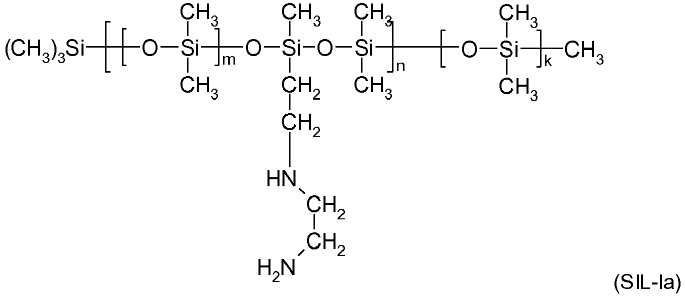

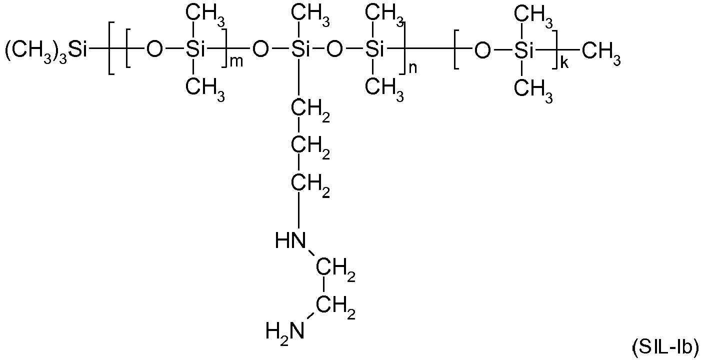

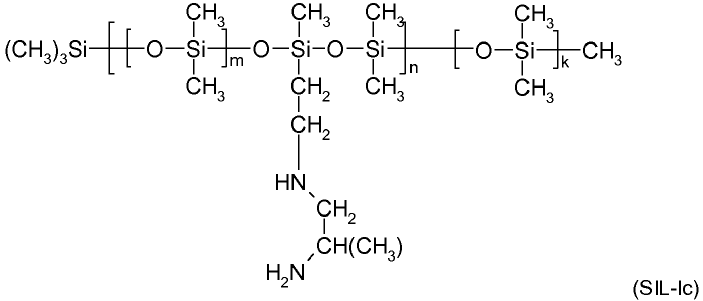

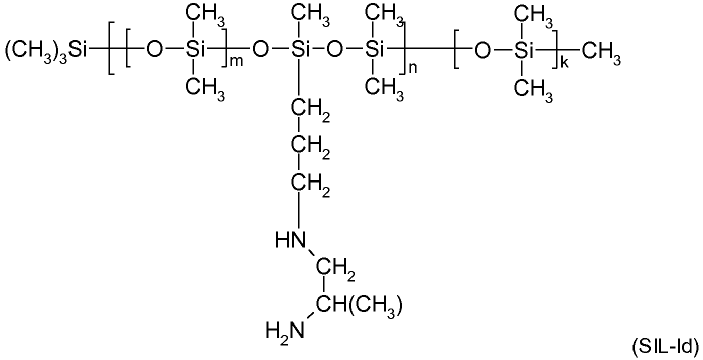

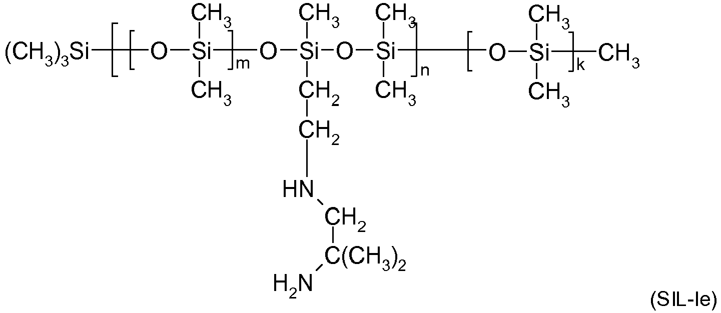

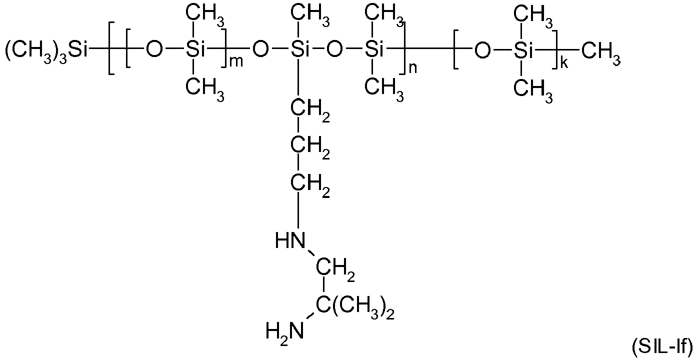

Siloxane der Formel (SIL-Ie), welche Aminoethylaminoisobutyl-Gruppen aufweisen, sind besonders bevorzugt.

Aus der Reaktion der organischen quaternären Ammoniumverbindung mit Epoxidgruppen oder Halohydringruppen, mit einem Silan oder Siloxan mit Aminogruppen gehen Verbindungen hervor, in denen ein Teil der Aminogruppen mit der Epoxygruppe oder der Halohydringruppe reagiert hat. Hierdurch wird die reagierende aminiofunktionelle Gruppe in eine Quat-Gruppe umgewandelt.

Aus den weiter oben genannten bevorzugten quaternären Ammoniumverbindungen mit Epoxidgruppen oder Halohydringruppen ergibt sich, daß bevorzugte erfindungsgemäße Haarbehandlungsmittel dadurch gekennzeichnet sind, daß das bzw. die elastomeren Silan(e) oder Siloxan(e) mit quaternären Ammoniumgruppen im Molekül als quaternäre Ammoniumgruppe die Gruppierung -CH₂CH(OH)CH₂N⁺(CH₃)₃ Cl⁻ aufweisen.

Aus der Reaktion der organischen quaternären Ammoniumverbindung mit Epoxidgruppen oder Halohydringruppen, mit einem Silan oder Siloxan mit Aminogruppen entstehen vorzugsweise Siloxane, die Einheiten des Typs 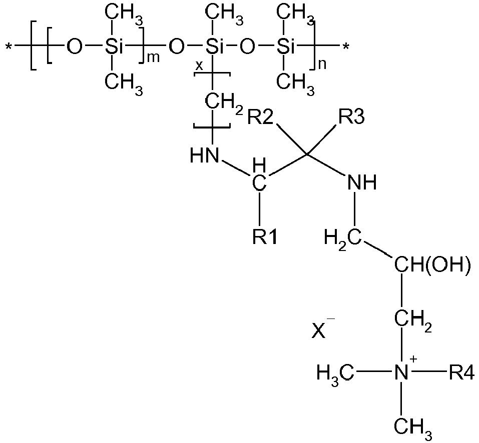 enthalten, wobei m und n sowie x sowie R¹, R² und R³ wie vorstehend definiert sind, X für Cl oder Br steht und R⁴ ein Alkylrest, vorzugsweise ein Methyl oder Dodecyl oder Octadecylrest ist.

Besonders bevorzugt gilt X = Cl und R⁴ = Methyl, so daß bevorzugte Siloxane Einheiten des Typs 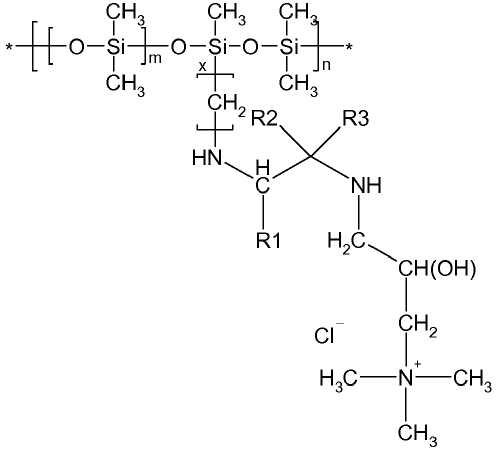 enthalten.

Berücksichtigt man, daß bei der Reaktion vorzugsweise nicht alle freien Aminogruppen abreagieren, werden Siloxane der Formel (SIL-II) erhalten: 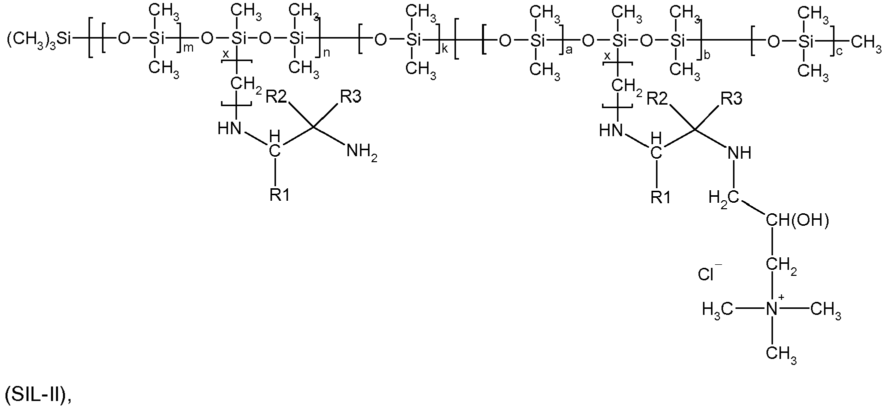 worin m, n, k, a, b, c so gewählt sind, daß das Siloxan 100 bis 5000 Si-Atome umfaßt. Die Indices stehen dabei vorzugsweise in einem solchen Verhältnis zueinander, daß die umgesetzten und unumgesetzten -(CH₂)ₓ-NH-CH(R¹)-C(R²)(R³)-NH₂ Gruppen 1 bis 5 Mol.-%, vorzugsweise 1,5 bis 4 Mol.-%, weiter bevorzugt 2 bis 3 Mol.-% und insbesondere 2 bis 2,5 Mol.-% ausmachen. Der Umsetzungsgrad, d.h. die Zahl der mit der organischen quaternären Ammoniumverbindung mit Epoxidgruppen oder Halohydringruppen, abreagierten -(CH₂)ₓ-NH-CH(R¹)-C(R²)(R³)-NH₂ Gruppen beträgt vorzugsweise 1 bis 50 % aller -(CH₂)ₓNH-CH(R¹)-C(R²)(R³)-NH₂ Gruppen, besonders bevorzugt 5 bis 40 % und insbesondere 10 bis 30 %.

Unter Berücksichtigung der bevorzugten Siloxane (SIL-Ia) bis (SIL-If) ergeben sich durch Reaktion mit der organischen quatemären Ammoniumverbindung mit Epoxidgruppen oder Halohydringruppen die bevorzugten Siloxane (SIL-IIa) bis (SIL-IIf): 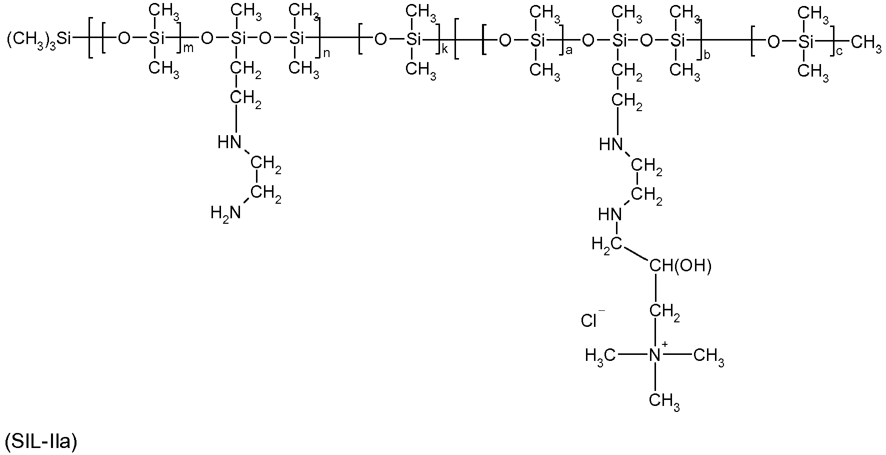 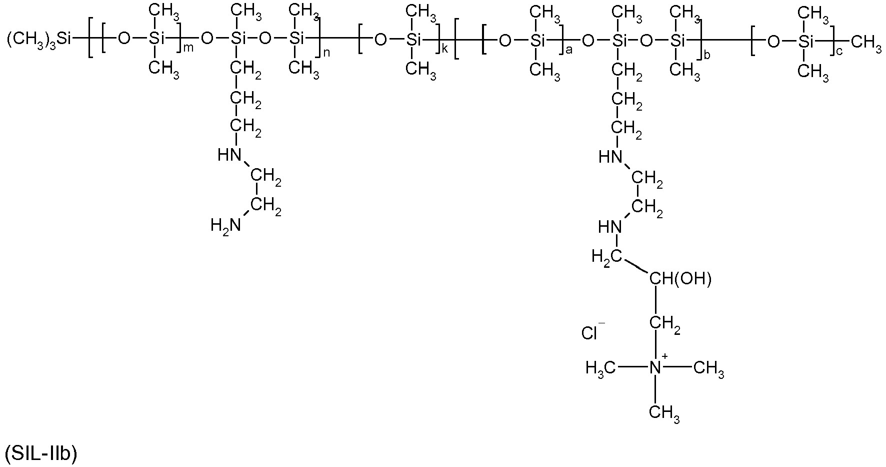 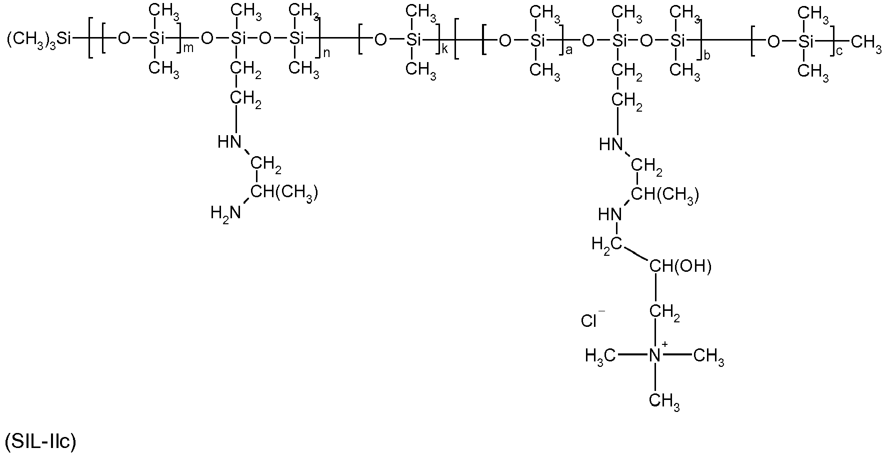 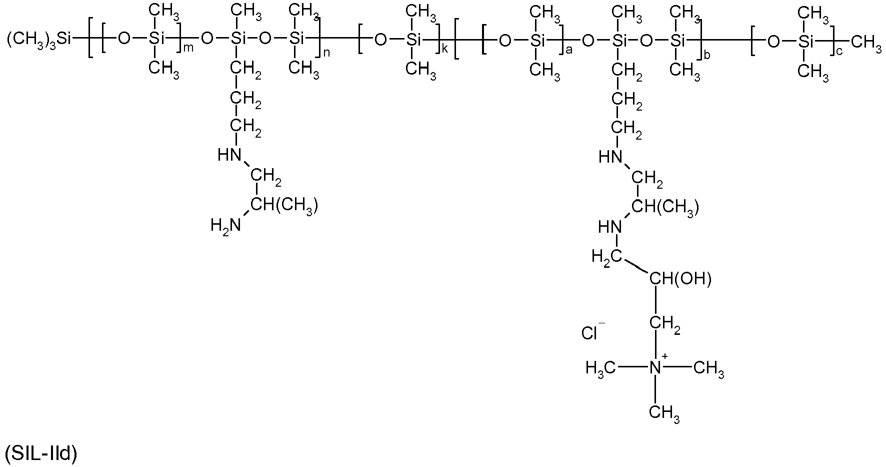 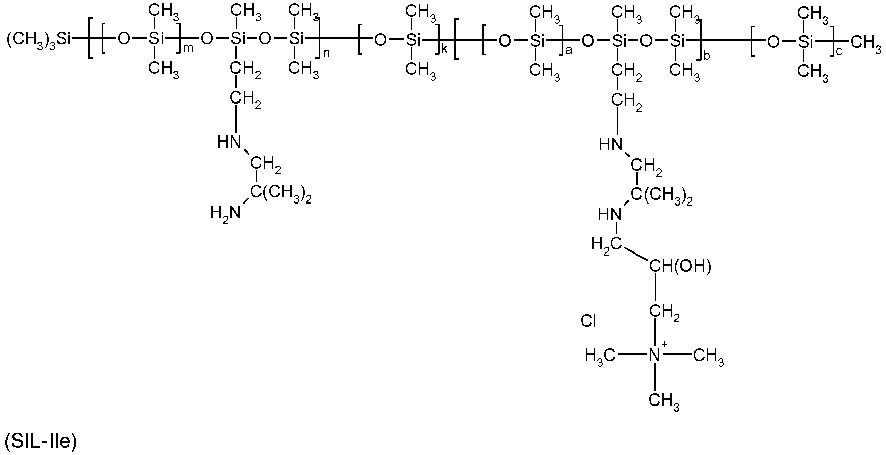 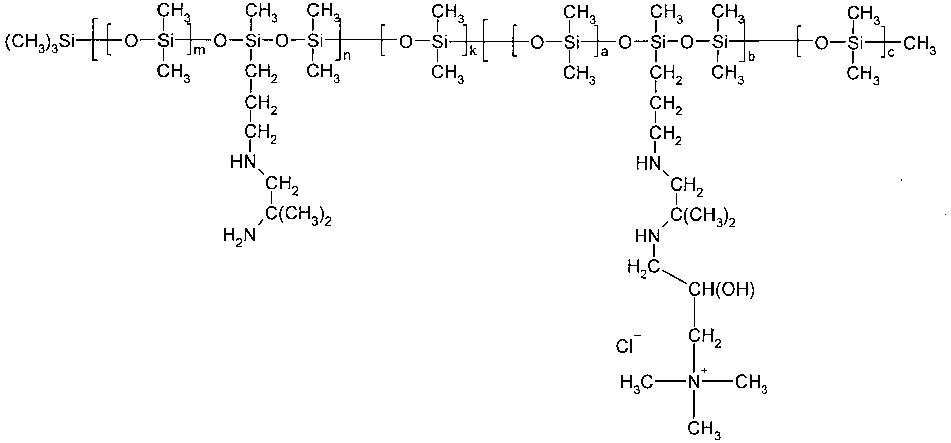 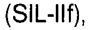 wobei wiederum Siloxane der Formel (SIL-IIe) bevorzugt sind.

In der Reaktionsmischung ist ein Verzweigungs-Agens aus der Gruppe
Butandioldiglycidether 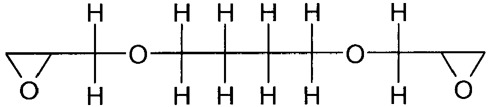 und Polypropylenglycoldiglycidether 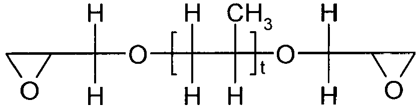 mit t = 1 bis 100, vorzugsweise 1 bis 50, enthalten.

Aus der Reaktion des Verzweigungsagens mit dem Silan oder Siloxan mit Aminogruppen gehen Verbindungen hervor, in denen ein Teil der Aminogruppen mit der Epoxygruppe oder der Halohydringruppe des Verzweigungsagens reagiert hat. Hierdurch wird die reagierende aminiofunktionelle Gruppe eines Silioxands mit der reagierenden aminofunktionellen Gruppe eines anderen Silioxans verbunden, so verzweigte Siloxane entstehen, die Einheiten des Typs 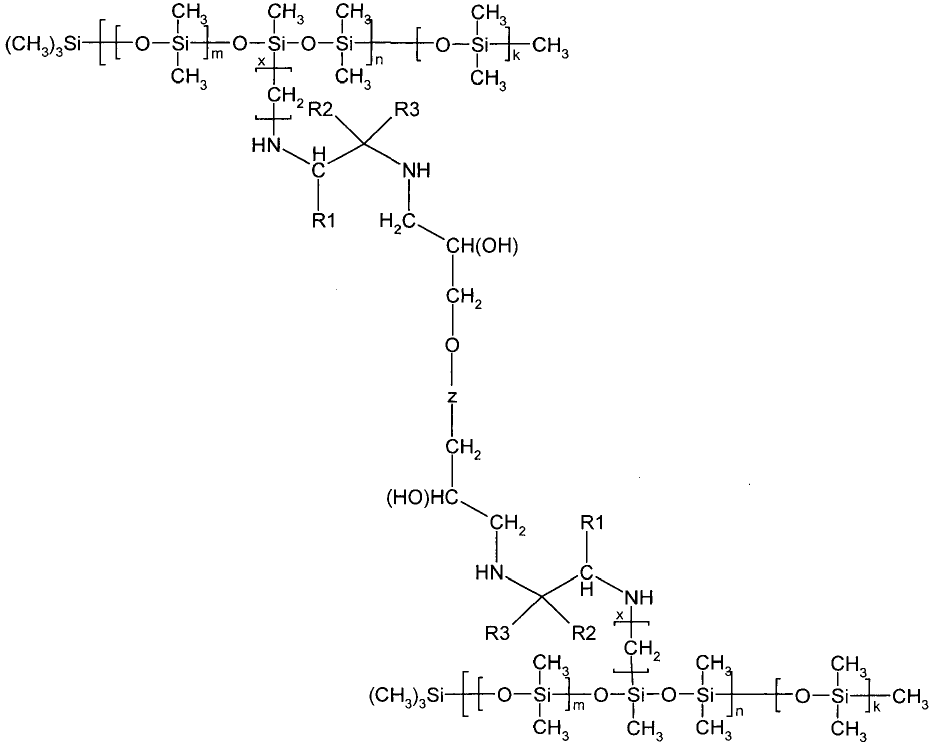 enthalten, wobei m und n sowie x sowie R¹, R² und R³ wie vorstehend definiert sind und Z für -(CH₂)₄-O- oder für-[CH₂-CH(CH₃)-O]ₜ- mit t = 1 bis 100, vorzugsweise 1 bis 50, steht.

Eine bevorzugte Einheit Z ist -(CH₂)₄-O-; wie weiter vorstehend erwähnt, ist x vorzugsweise 2, R1 ist vorzugsweise -H, R2 und R3 sind vorzugsweise Methyl.

Da ein Teil der Aminogruppen des Siloxans umgesetzt bleibt, ein anderer Teil mit dem Verzweigungsagens reagiert, während ein weiterer Teil mit der organischen quaternären Ammoniumverbindung reagiert, entstehen verzweigten Siloxan-Crosspolymere mit Aminofunktionellen Gruppe und mit Quatgruppen. Der Einsatz dieser Silikon-Elastomere in den erfindungsgemäßen Haarbehandlungsmittel ist bevorzugt. Besonders bevorzugt werden erfindungsgemäß Silikonelastomere eingesetzt, die sich durch die nachstehende Formel beschreiben lassen:

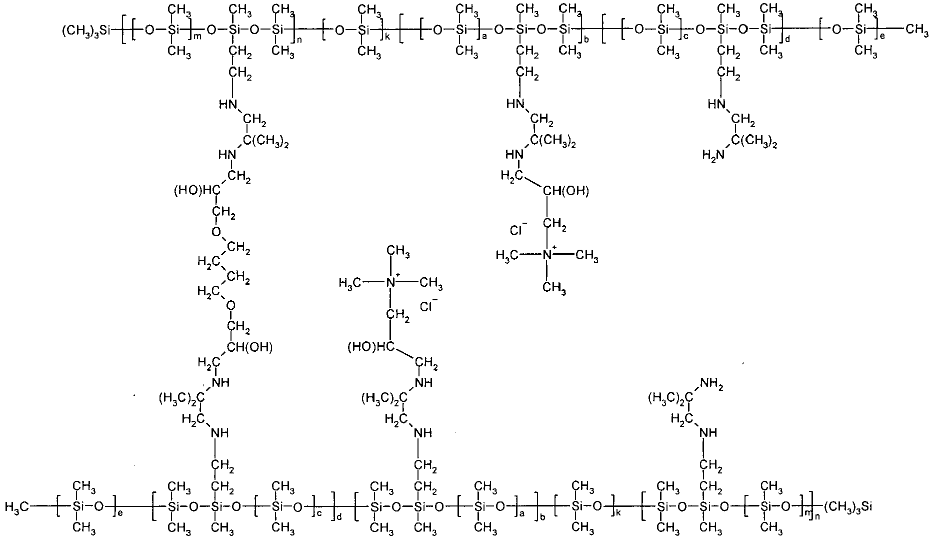

In der Reaktionsmischung ist weiterhin mindestens ein Tensid enthalten, so daß die spätere Emulsion des Silikonelastomers ebenfalls Tensid enthält. Das in der Reaktionsmischung enthaltene Tensid ist ausgewählt aus ethoxylierten Alkoholen der Formel

CₙH₂ₙ₊₁(OCH₂CH₂)ₓOH

in der n für Zahlen zwischen 8 und 20, vorzugsweise für 13, und x für Zahlen von 8 bis 18, vorzugsweise für 12, stehen.

Die wäßrige polare Phase der Silikonelastomeremulsion enthält Wasser. Zusätzlich können nichtwäßrige polare Solventien enthalten sein, beispielsweise Ethanol, n-Propanol, Isopropanol, Diole wie Ethylenglycol, Propylenglycol, 1,6-Hexandiol, 2-Methyl-1,3-Propandiol, Triole wie Glycerin Ester wie Triacetin und Glycerintripropionat usw.. Vorzugsweise enthält die wäßrige Silikonlastomeremulsion, die in den erfindungsgemäßen Haarbehandlungsmitteln eingesetzt wird, bezogen auf ihr Gewicht 20 bis 90 Gew.-%, vorzugsweise 30 bis 80 Gew.-% und insbesondere 50 bis 70 Gew.-% Wasser.

Die erfindungsgemäßen Mittel können weitere Wirk- und Hilfsstoffe beinhalten. Diese werden nachfolgend beschrieben.

Vorzugsweise enthalten die erfindungsgemäßen Mittel zusätzlich mindestens einen Emulgator bzw. ein Tensid, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind.

Erfindungsgemäß bevorzugte Haarbehandlungsmittel sind dadurch gekennzeichnet, daß sie - bezogen auf sein Gewicht - 0,5 bis 70 Gew.-%, vorzugsweise 1 bis 60 Gew.-% und insbesondere 5 bis 25 Gew.-% anionische(s) und/oder nichtionische(s) und/oder kationische(s) und/oder amphotere(s) Tensid(e), enthalten.

Als anionische Tenside und Emulgatoren eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓCH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Acylglutamate der Formel (I), 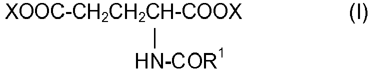 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 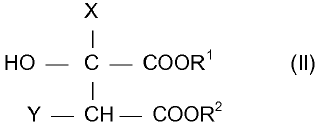 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobernsteinsäure oder der Sulfosuccinate der allgemeinen Formel (III), 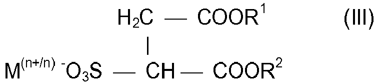 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)_{X} OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside und Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.

Als zwitterionische Tenside und Emulgatoren werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈- C₂₄- Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside und Emulgatoren enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie Hydagen® HSP (Cognis) oder Sovermol® Typen (Cognis), insbesondere von gesättigten C₈₋₃₀-Fettsäuren,
- alkoxylierte Triglyceride,
- alkoxylierte Fettsäurealkylester,
- Aminoxide,
- Fettsäurealkanolamide, Fettsäure-N-alkylglucamide und Fettamine sowie deren Ethylenoxid- oder Polyglycerin-Anlagerungsprodukte,
- Sorbitanfettsäureester und Anlagerungsprodukte von Ethylenoxid an Sorbitanfettsäureester wie beispielsweise die Polysorbate,
- Zuckerfettsäureester und Methylglucosid-Fettsäureester sowie deren Ethylenoxid- oder Polyglycerin-Anlagerungsprodukte,
- Alkylpolyglycoside entsprechend der allgemeinen Formel RO-(Z)ₓ wobei R für Alkyl, Z für Zucker sowie x für die Anzahl der Zuckereinheiten steht.

Besonders bevorzugt sind solche Alkylpolyglycoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen
besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.
- Gemische aus Alkyl-(oligo)-glucosiden und Fettalkoholen, z. B. Montanov®68,
- Sterine, z. B. Ergosterin, Stigmasterin, Sitosterin und Mykosterine,
- Phospholipide, z. B. Lecithine bzw. Phosphatidylcholine,
- Polyglycerine und Polyglycerinderivate wie beispielsweise Polyglycerinpoly-12-hydroxystearat (Dehyrnuls® PGPH) oder Triglycerindüsostearat (Lameform® TGI),
- alkoxylierte Polydialkylsiloxane (INCI-Bezeichnung: Dimethicone Copolyol).

Als bevorzugte nichtionische oberflächenaktive Substanzen haben sich die Alkylpolyglycoside, gegebenenfalls im Gemisch mit Fettalkoholen, alkoxylierte Polydialkylsiloxane, Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Ganz besonders bevorzugt sind erfindungsgemäße Mittel, die zusätzlich Fettalkohol(e) und/oder Fettalkoholalkoxylat(e), vorzugsweise C₁₂₋₂₂-Fettalkohol(e) und/oder C₁₂₋₂₂-Fettalkoholethoxylat(e) mit 10 bis 30 EO-Einheiten, besonders bevorzugt C₁₆₋₁₈-Fettalkohol(e) und/oder C₁₆₋₁₈-Fettalkoholethoxylat(e) mit 12 bis 20 EO-Einheiten, vorzugsweise in Mengen von 5 bis 20 Gew.-%, bevorzugt von 7,5 bis 17,5 Gew.-% und insbesondere von 10 bis 15 Gew.-%, jeweils bezogen auf das Gewicht des Mittels, enthalten.

Zusammenfassend sind erfindungsgemäße Haarbehandlungsmittel bevorzugt, die - bezogen auf ihr Gewicht - 0,1 bis 20 Gew.-%, vorzugsweise 0,25 bis 17,5 Gew.-% und insbesondere 5 bis 15 Gew.-% anionische(s) Tensid(e), besonders bevorzugt Fettalkoholethersulfate der Formel

**H₃C-(CH₂)ₙ-(OCH₂CH₂)ₖ-OSO₃⁻ M⁺**

enthalten, in der n für Werte von 5 bis 21, vorzugsweise von 7 bis 19, besonders bevorzugt von 9 bis 17 und insbesondere von 11 bis 13 und k für Werte von 1, 2, 3, 4, 5, 6, 7, 8, 9 oder 10, vorzugsweise für 1, 2 oder 3 und insbesondere für 2 stehen, und M für ein Kation aus der Gruppe Na⁺, K⁺ NH₄⁺, ½ Mg²⁺, ½ Zn²⁺, vorzugsweise für Na⁺, stehen.

Bevorzugte erfindungsgemäße Haarbehandlungsmittel sind weiter dadurch gekennzeichnet, daß sie zusätzlich amphotere(s) Tensid(e), vorzugsweise aus den Gruppen der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren mit jeweils etwa 8 bis 24 C-Atomen in der Alkylgruppe, Alkylaminoessigsäuren mit jeweils etwa 8 bis 24 C-Atomen in der Alkylgruppe, N-Kokosalkylaminopropionat, Kokosacylaminoethylaminopropionat C₁₂ - C₁₈ - Acylsarcosin, N-Alkyl-N,N-dimethylammonium-glycinate, beispielsweise Kokosalkyl-dimethylammoniumglycinat, N-Acyl-aminopropyl-N,N-dimethylammoniumglycinate, beispielsweise Kokosacylaminopropyl-dimethylammoniumglycinat, 2-Alkyl-3-carboxymethyl-3-hydroxyethyl-imidazoline mit jeweils 8 bis 18 C-Atomen in der Alkyl- oder Acylgruppe, Kokosacylaminoethylhydroxyethylcarboxymethylglycinat, der unter der INCI-Bezeichnung Cocamidopropyl Betain bekannten Verbindungen, der unter der INCI-Bezeichnung Disodium Cocoamphodiacetate bekannten Verbindungen, enthalten, wobei bevorzugte Mittel das bzw. die amphotere(n) Tensid(e) in Mengen von 1 bis 15 Gew.-%, vorzugsweise von 2,5 bis 12 Gew.-% und insbesondere von 5 bis 10 Gew.-%, jeweils bezogen auf das gesamte Mittel, enthalten.

Als weiteren optionalen Bestandteil können die erfindungsgemäßen Mittel 0,01 bis 10 Gew.-% mindestens eines Polymers aus der Gruppe der kationischen und/oder amphoteren Polymere enthalten.

Unter kationischen bzw. amphoteren Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Zusätzlich zu kationischen Polymerisaten oder an ihrer Stelle können die erfindungsgemäßen Mittel auch amphotere Polymere enthalten. Diese weisen zusätzlich mindestens eine negativ geladene Gruppe im Molekül auf und werden auch als zwitterionische Polymere bezeichnet.

Vorzugsweise wird das Polymer bzw. werden die Polymere innerhalb engerer Mengenbereiche eingesetzt. So sind erfindungsgemäße Mittel bevorzugt, die - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% amphotere(s) Polymer(e), enthalten.

Unabhängig davon, ob in den Mitteln amphotere Polymere enthalten sind oder nicht, sind weiter bevorzugte erfindungsgemäße Mittel dadurch gekennzeichnet, daß sie - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Polymer(e), enthalten.

Erfindungsgemäß bevorzugt einsetzbare kationische Polymere werden nachstehend beschrieben: Homopolymere der allgemeinen Formel (G1-I), 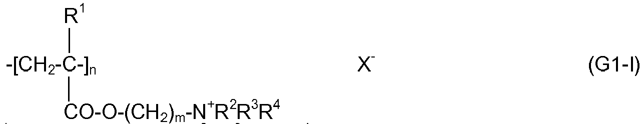 in der R¹= -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare® CTH (Cosmetic Rheologies) und Synthalen® CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vemetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Als kationische Polymere können auch kationische Proteinhydrolysate eingesetzt werden, wobei bevorzugte Mittel ein oder mehrere kationische Proteinhydrolysate aus der Gruppe Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein enthalten.

Zusammenfassend sind erfindungsgemäße Haarbehandlungsmittel bevorzugt, die - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Polymer(e), enthalten. Bevorzugte kationische(s) Polymer(e) ist/sind ausgewählt aus Poly(methacryloyloxyethyltrimethyl-ammoniumchlorid) (INCI: Polyquatemium-37) und/oder quaternisierten Cellulose-Derivaten (INCI: Polyquaternium 10) und/oder kationischen Alkylpolyglycosiden und/oder kationisiertem Honig und/oder kationischen Guar-Derivaten und/oder polymeren Dimethyldiallylammoniumsalzen und deren Copolymeren mit Estern und Amiden von Acrylsäure und Methacrylsäure und/oder Copolymeren des Vinylpyrrolidons mit quaternierten Derivaten des Dialkylaminoalkylacrylats und - methacrylats und/oder Vinylpyrrolidon-Vinylimidazoliummethochlorid-Copolymeren und/oder quaterniertem Polyvinylalkohol und/oder Polyquaternium 2 und/oder Polyquaternium-7 und/oder Polyquaternium 17 und/oder Polyquaternium 18 und/oder Polyquaternium 24 und/oder Polyquaternium 27.

Zusätzlich zu den kationischen Polymeren oder an ihrer Stelle können die erfindungsgemäßen Mittel amphotere Polymere enthalten. Im Rahmen der vorliegenden Erfindung bevorzugt einsetzbare amphotere Polymerisate setzen sich im wesentlichen zusammen aus
A) Monomeren mit quartären Ammoniumgruppen der allgemeinen Formel (Z-I),

   R¹-CH=CR²-CO-Z-(CₙH₂ₙ)-N⁽⁺⁾R³R⁴R⁵ A⁽⁻⁾ (Z-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
B) monomeren Carbonsäuren der allgemeinen Formel (Z-II),

   R⁶-CH=CR⁷-COOH (Z-II)

   in denen R⁶ und R⁷ unabhängig voneinander Wasserstoff oder
   Methylgruppen sind.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quatemisierungsreaktion kann in wäßriger Lösung oder im Lösungsmittel erfolgen.

Vorteilhafterweise werden solche Monomere der Formel (Z-I), die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (Z-I), bei denen R³, R⁴ und R⁵ Methylgruppen sind.

Das Acrylamidopropyl-trimethylammoniumchlorid ist ein ganz besonders bevorzugtes Monomer der Formel (Z-I).

Als monomere Carbonsäuren der Formel (Z-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (Z-I) und (Z-II) nach an sich bekannten Polymerisationsverfahren hergestellt. Die Polymerisation kann entweder in wäßriger oder wäßrig-alkoholischer Lösung erfolgen. Als Alkohole werden Alkohole mit 1 bis 4 Kohlenstoffatomen, vorzugsweise Isopropanol, verwendet, die gleichzeitig als Polymerisationsregler dienen. Der Monomerlösung können aber auch andere Komponenten als Regler zugesetzt werden, z. B. Ameisensäure oder Mercaptane, wie Thioethanol und Thioglykolsäure. Die Initiierung der Polymerisation erfolgt mit Hilfe von radikalbildenden Substanzen. Hierzu können Redoxsysteme und/oder thermisch zerfallende Radikalbildner vom Typ der Azoverbindungen, wie z. B. Azoisobuttersäurenitril, Azo-bis-(cyanopentansäure) oder Azo-bis-(amidinopropan)dihydrochlorid verwendet werden. Als Redoxsysteme eignen sich z. B. Kombinationen aus Wasserstoffperoxid, Kalium- oder Ammoniumperoxodisulfat sowie tertiäres Butylhydroperoxid mit Natriumsulfit, Natriumdithionit oder Hydroxylaminhydrochlorid als Reduktionskomponente.

Die Polymerisation kann isotherm oder unter adiabatischen Bedingungen durchgeführt werden, wobei in Abhängigkeit von den Konzentrationsverhältnissen durch die freiwerdende Polymerisationswärme der Temperaturbereich für den Ablauf der Reaktion zwischen 20 und 200 °C schwanken kann, und die Reaktion gegebenenfalls unter dem sich einstellenden Überdruck durchgeführt werden muß. Bevorzugterweise liegt die Reaktionstemperatur zwischen 20 und 100 °C.

Der pH-Wert während der Copolymerisation kann in einem weiten Bereich schwanken. Vorteilhafterweise wird bei niedrigen pH-Werten polymerisiert; möglich sind jedoch auch pH-Werte oberhalb des Neutralpunktes. Nach der Polymerisation wird mit einer wäßrigen Base, z. B. Natronlauge, Kalilauge oder Ammoniak, auf einen pH-Wert zwischen 5 und 10, vorzugsweise 6 bis 8, eingestellt. Nähere Angaben zum Polymerisationsverfahren können den Beispielen entnommen werden.

Als besonders wirksam haben sich solche Polymerisate erwiesen, bei denen die Monomeren der Formel (Z-I) gegenüber den Monomeren der Formel (Z-II) im Überschuß vorlagen. Es ist daher erfindungsgemäß bevorzugt, solche Polymerisate zu verwenden, die aus Monomeren der Formel (Z-I) und die Monomeren der Formel (Z-II) in einem Molverhältnis von 60:40 bis 95:5, insbesondere von 75:25 bis 95:5, bestehen.

Als weiteren Inhaltsstoff können die erfindungsgemäßen Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte Haarbehandlungsmittel dadurch gekennzeichnet, daß sie als Pftegestoff- bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Eine weitere bevorzugte Gruppe von Inhaltsstoffen der erfindungsgemäßen Mittel sind Vitamine, Provitamine oder Vitaminvorstufen. Es sind erfindungsgemäße Haarbehandlungsmittel bevorzugt, die zusätzlich als Pflegestoff - bezogen auf sein Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel Panthenol ((±)-2,4-Dihydroxy-*N*-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, daß der Einsatz bestimmter Chinone eine Antischuppen- und Anti-Haarausfallwirkung verstärkt sowie Vorteile in Bezug auf Kämmbarkeit und Glanz bewirkt. Als weiteren Bestandteil können die erfindungsgemäßen Mittel daher 0,0001 bis 5 Gew.-% mindestens eines Biochinons der Formel (I) 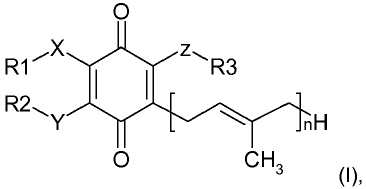 enthalten in der
- X, Y, Z: stehen unabhängig voneinander für -O- oder -NH- oder NR⁴- oder eine chemische Bindung
- R¹, R², R³: stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, -CH₃, -CH₂CH₃, -(CH₂)₂CH₂,-CH(CH₃)₂, -(CH₂)₃CH₃, -CH(CH₃)CH₂CH₃, -CH₂CH(CH₃)₂, -C(CH₃)₃
R⁴ steht für -CH₃, -CH₂CH₃, -(CH₂)₂CH₂, -CH(CH₃)₂, -(CH₂)₃CH₃, -CH(CH₃)CH₂CH₃,-CH₂CH(CH₃)₂, -C(CH₃)₃
n steht für Werte von 1 bis 20, vorzugsweise von 2 bis 15 und insbesondere für 5, 6, 7, 8, 9, 10. Besonders bevorzugte erfindungsgemäße Haarbehandlungsmittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 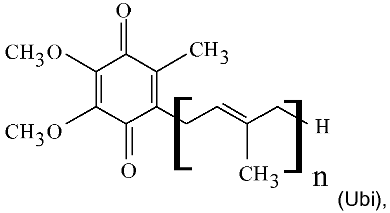 in der n für die Werte = 6, 7, 8, 9 oder 10, besonders bevorzugt für 10 (Coenzym Q10) steht.

Zur Verbesserung der Elastizität und Festigung der inneren Struktur der mit erfindungsgemäßen Mitteln behandelter Haare können die erfindungsgemäßen Mittel Purin und/oder Purinderivate enthalten. Insbesondere die Kombination von Purin und/oder Purinderivaten mit Ubichinonen und/oder Plastochinonen führt dazu, daß die mit entsprechenden Mitteln behandelten Haare unter anderem höhere Meßwerte bei der Differenzthermoanalyse und verbesserte Naß- und Trockenkämmbarkeiten zeigen.

Als weiteren Inhaltsstoff können die erfindungsgemäßen Mittel daher Purin und/oder Derivat(e) des Purins enthalten. Purin (*7H*-Imidazo[4,5-*d*]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten.

Bevorzugte erfindungsgemäße Mitteln enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß einige Vertreter besonders bevorzugt. Erfindungsgemäß bevorzugte Haarbehandlungsmittel sind dadurch gekennzeichnet, daß sie als Pflegestoff - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten 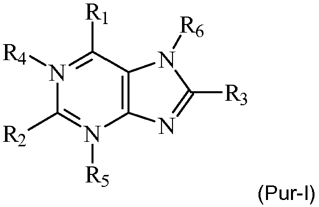 in der die Reste R¹, R² und R³ unabhängig voneinander ausgewählt sind aus -H, - OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind: Purin (R¹ = R² = R³ = R⁴ = R⁵ = R⁶ = H), Adenin (R¹ = NH₂, R² = R³ = R⁴ = R⁵ = R⁶ = H), Guanin (R¹ **=** OH, R² = NH₂, R³ = R⁴ = R⁵ = R⁶ = H), Harnsäure (R¹ = R² = R³ = OH, R⁴ = R⁵ = R⁶ = H), Hypoxanthin (R¹ = OH, R² = R³ = R⁴ = R⁵ = R⁶ = H), 6-Purinthiol (R¹ = SH, R² = R³ = R⁴ = R⁵ = R⁶ = H), 6-Thioguanin (R¹ = SH, R² = NH₂, R³ = R⁴ = R⁵ = R⁶ = H), Xanthin (R¹ = R² = OH, R³ = R⁴ = R⁵ = R⁶ = H), Coffein (R¹ = R² = OH, R³ = H, R⁴ = R⁵ = R⁶ = CH₃), Theobromin (R¹ = R² = OH, R³ = R⁴ = H, R⁵ = R⁶ = CH₃), Theophyllin (R¹ = R² = OH, R³ = H, R⁴ = ^{CH}₃, R⁵ = CH₃, R⁶ = H).

Je nach gewünschtem Anwendungszweck der erfindungsgemäßen Mittel kann dabei die Art und Menge des Purinderivates variieren. In haarkosmetischen Formulierungen hat sich insbesondere Coffein bewährt, das beispielsweise in Shampoos vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf das Shampoo) eingesetzt werden kann.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haarbehandlungsmittel dadurch gekennzeichnet, daß sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus Monosachhariden (insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose), Disacchariden (insbesondere Saccharose und/oder Maltose und/oder Lactose und/oder Trehalose und/oder Cellobiose und/oder Gentiobiose und/oder Isomaltose).

Die erfindungsgemäßen Mittel können zusätzlich zu dem/den Chitosan(derivat)(en) und dem bzw. den Silikonelastomeren sowie optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft. Diese Stoffe werden nachstehend beschrieben: Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, daß sich weniger DHT aus Testosteron bilden kann. Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältlich. Zur Bekämpfung der hormonellen Einflüße auf die Haarfollikel kann zur äußeren Anwendung Spironolactone in Form von Haarwasser und in Kombination mit Minoxidil angewandt werden. Spironolactone wirkt als Androgen-Rezeptor-Blocker, dh. die Bindung von DHT an die Haarfollikel wird verhindert.

Zusammenfassend sind erfindungsgemäße kosmetische Mittel bevorzugt, die zusätzlich - bezogen auf sein Gewicht - 0,001 bis 5 Gew.-% Haarwurzel-stabilisierende Stoffe, insbesondere Minoxidil und/oder Finasterid und/oder Ketoconazol enthalten.

Durch zusätzliche Antischuppenwirkstoffe (beispielsweise Climbazol, Piroctone Olamine oder Zink-Pyrithion) wird die Menge des Schuppen verursachenden Hefepilzes gezielt reduziert, die Keimflora erreicht wieder die normale prozentuale Zusammensetzung und die Abschuppung wird auf das physiologische Maß reduziert. Labortests haben jedoch nachgewiesen, daß die unterschiedlichen Artvertreter des Pityrosporum ovale unterschiedlich gut auf die Antischuppenwirkstoffe reagieren. Um alle Schuppenerreger maximal zu bekämpfen ist daher eine Kombination von Anti-Schuppenwirkstoffen am erfolgreichsten.

Zusammenfassend sind erfindungsgemäße Haarbehandlungsmittel bevorzugt, die zusätzlich - bezogen auf ihr Gewicht - 0,001 bis 5 Gew.-% Antischuppenwirkstoffe, insbesondere Piroctone Olamine (1-Hydroxy-4-methyl-6-(2,4,4-trirnethylpentyl)pyridin-2(1H)-on, Verbindung mit 2-Aminoethanol, 1:1) und/oder Zink-Pyrithion und/oder Selensulfid und/oder Climbazol und/oder Salicylsäure oder Fumarsäure enthalten.

Die erfindungsgemäßen Mittel können weiterhin alle für solche Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Diese Tenside wurden weiter oben ausführlich beschrieben.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn zusätzlich zu dem bzw. den Polymer(en) aus der Gruppe der kationischen und/oder amphoteren Polymere weitere Polymere (G) in den erfindungsgemäßen Mitteln enthalten sind. In einer bevorzugten Ausführungsform werden den erfindungsgemäßen Mitteln daher weitere Polymere zugesetzt, wobei sich sowohl anionische als auch nichtionische Polymere als wirksam erwiesen haben.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vemetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vemetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vemetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Die erfindungsgemäßen Mittel können in einer weiteren Ausführungsform nichtionogene Polymere (G4) enthalten.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) und Natrosol®-Typen (Hercules) vertrieben werden.
- Stärke und deren Derivate, insbesondere Stärkeether, beispielsweise Structure® XL (National Starch), eine multifunktionelle, salztolerante Stärke;
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten.

Die weiteren Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestem, Diphenylacrylsäureestern, Zimtsäureestern, Salicylsäureestern, Benzimidazolen und o-Aminobenzoesäureestern.

Die erfindungsgemäßen Mittel können weiterhin eine 2-Pyrrolidinon-5-carbonsäure und deren Derivate (J) enthalten. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.%.

Schließlich können die erfindungsgemäßen Mittel auch Pflanzenextrakte (L) enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt. Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Eine besonders bevorzugte Gruppe von Inhaltsstoffen stellen die Silikone dar.

Erfindungsgemäße bevorzugte Mittel sind dadurch gekennzeichnet, daß sie mindestens ein Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter: substituierten oder unsubstituierten aminierten Gruppen; (per)fluorierten Gruppen; Thiolgruppen; Carboxylatgruppen; hydroxylierten Gruppen; alkoxylierten Gruppen; Acyloxyalkylgruppen; amphoteren Gruppen; Bisulfitgruppen; Hydroxyacylaminogruppen; Carboxygruppen; Sulfonsäuregruppen; und Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
oder deren Gemischen.

Erfindungsgemäß besonders bevorzugte Mittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Bevorzugte Silikone werden nachstehend beschrieben.

Besonders bevorzugte erfindungsgemäße Mittel sind dadurch gekennzeichnet, daß sie mindestens ein Silikon der Formel Si-I

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Diese Silikone werden nach der INCI-Nomenklatur als DIMETHICONE bezeichnet.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Besonders bevorzugte erfindungsgemäße Mittel enthalten ein oder mehrere aminofunktionelle Silicone. Solche Silicone können z.B. durch die Formel

M(RₐQ_{b}SiO_{(4-a-b)/2)x}(RcSiO_{(4-c)/2)y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen,-CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH ₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Mittel sind dadurch gekennzeichnet, daß sie ein aminofunktionelles Silikon der Formel (Si-II)

R'ₐG₃₋ₐ-Si(OSiG₂)ₙ-(OSiG_{b}R'_{2-b})ₘ-O-SiG₃₋ₐ-R'ₐ (Si-II),

enthalten, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂,-CH₂CH(CH₃)₂, -O-CH(CH₃)CH₂CH₃, -CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂A⁻
   ∘ -Q-N⁺H₂(R")A⁻
   ∘ -Q-N(R")-CH₂-CH₂-N⁺R"H₂A⁻,
   wobei jedes Q für eine chemische Bindung, -CH₂-, -CH₂-CH₂-, -CH₂CH₂CH₂-, -C(CH₃)₂-,-CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃,-CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, daß sie mindestens ein aminofunktionelles Silikon der Formel (Si-IIa) 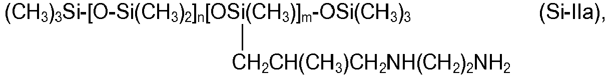 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch erfindungsgemäße Mittel, die ein aminofunktionelles Silikon der Formel (Si-IIb) 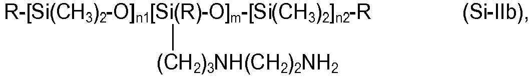 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Erfindungsgemäß bevorzugte Mittel sind dadurch gekennzeichnet, daß sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als CYCLOMETHICONE bezeichneten cyclischen Dimethicone sind erfindungsgemäß mit Vorzug einsetzbar. Hier sind erfindungsgemäße Mittel bevorzugt, die mindestens ein Silikon der Formel Si-III 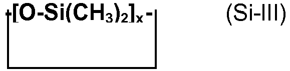 enthalten, in der x für eine Zahl von 3 bis 200, vorzugsweise von 3 bis 10, weiter bevorzugt von30 bis 7 und insbesondere 3, 4, 5 oder 6, steht.

Die vorstehend beschriebenen Silicone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Erfindungsgemäß ebenfalls bevorzugte Mittel sind dadurch gekennzeichnet, daß sie mindestens ein Silikon der Formel Si-IV

R₃Si-[O-SiR₂]ₓ-(CH₂)ₙ-[O-SiR₂]_{y}-O-SiR₃ (Si-IV),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Mittel sind dadurch gekennzeichnet, daß sie mindestens ein wasserlösliches Silikon enthalten.

Aus ästhetischen Gründen werden "klare" Produkte von Verbrauchern oft bevorzugt. Erfindungsgemäß bevorzugte Haarbehandlungsmittel sind daher dadurch gekennzeichnet, daß sie transparent bzw. transluzent sind.

Unter transparent oder transluzent wird im Rahmen der vorliegenden Erfindung eine Zusammensetzung verstanden, die einen NTU-Wert von unter 100 aufweist. Der NTU-Wert (Nephelometric Turbidity Unit, *Nephelometrischer Trübungswert;* NTU) ist eine in der Wasseraufbereitung verwendete Einheit für Trübungsmessungen in Flüssigkeiten. Sie ist die Einheit einer mit einem kalibriertem Nephelometer gemessenen Trübung einer Flüssigkeit.

Die erfindungsgemäßen Mittel weisen vorteilhafte Eigenschaften auf und verleihen den mit ihnen behandelten Haaren ebenfalls vorteilhafte Eigenschaften. Insbesondere bei der Haar- und Kopfhautbehandlung wurden Vorteile beobachtet. So steigern erfindungsgemäße Haarbehandlungsmittel die Elastizität der mit ihnen behandelten Haare und führen zu einer innerstrukturellen Stärkung der Haarfasern, welche sich z.B. in höheren Schmelztemperaturen bei der Differenzthermoanalyse, einem deutlich gesteigerten Volumen und einem höheren Frisurenhalt über längere Zeiträume niederschlägt.

Es zeigt sich auch eine Verbesserung der Naß- und Trockenkämmbarkeiten sowie eine Verhinderung frühzeitiger Splißbildung bei den behandelten Haaren.

Ein weiterer Gegenstand der vorliegenden Erfindung ist die Verwendung von Mischungen aus
a) mindestens einem Chitosan und/oder Chitosanderivat und
b) mindestens einem elastomeren Silan oder Siloxan mit quaternären Ammoniumgruppen im Molekül
zur Verbesserung mindestens einer der Eigenschaften
- Zugfestigkeit von keratinischen Fasern, insbesondere menschlichen Haaren;
- Stabilisierung des Feuchtigkeitshaushaltes von keratinischen Fasern, insbesondere menschlichen Haaren;
- Kämmbarkeit von keratinischen Fasern, insbesondere menschlichen Haaren;
- Steigerung des Volumens von keratinischen Fasern, insbesondere menschlichen Haaren;
- Verzögerung des Alterungsprozesses von keratinischen Fasern, insbesondere menschlichen Haaren;
- Verringerung der Elastizitätsabnahme von keratinischen Fasern, insbesondere menschlichen Haaren bei Beschädigung durch atmosphärische Einwirkungen.

Diese Eigenschaften besitzen durch den Einsatz der speziellen Wirkstoffkombination auch die erfindungsgemäßen Mittel. Ein weiterer Gegenstand der vorliegenden Erfindung ist daher die Verwendung von erfindungsgemäßen Haarbehandlungsmittel zur Verbesserung mindestens einer der Eigenschaften
- Zugfestigkeit von keratinischen Fasern, insbesondere menschlichen Haaren;
- Stabilisierung des Feuchtigkeitshaushaltes von keratinischen Fasern, insbesondere menschlichen Haaren;
- Kämmbarkeit von keratinischen Fasern, insbesondere menschlichen Haaren;
- Steigerung des Volumens von keratinischen Fasern, insbesondere menschlichen Haaren;
- Verzögerung des Alterungsprozesses von keratinischen Fasern, insbesondere menschlichen Haaren;
- Verringerung der Elastizitätsabnahme von keratinischen Fasern, insbesondere menschlichen Haaren bei Beschädigung durch atmosphärische Einwirkungen.

Bezüglich weiterer bevorzugter Ausführungsformen der erfindungsgemäßen Verwendung gilt mutatis mutandis das zu den erfindungsgemäßen Mitteln Gesagte.

### Beispiel

Die folgenden Mengenangaben verstehen sich - soweit nichts anderes vermerkt ist - in Gewichtsprozent.

Es wurde nachfolgende Rezeptur hergestellt.

| **Rohstoffe** | |
|---|---|
| Benzophenone-4 | 0,05 |
| Zitronensäure | 0,01 |
| Kytamer PC ¹ | 1,00 |
| Ethanol | 15,00 |
| Dow Corning 5-7070 Emulsion ² | 5,00 |
| D-Panthenol | 0,25 |
| PEG-40 Hydrogenated Castor Oil | 0,50 |
| Parfum | 0,10 |
| Wasser | ad 100 |

| | |
|---|---|
| ¹ Chitosoniumpyrrolidoncarboxylat, Reaktionsprodukt von Chitosan mit Pyrrolidoncarbonsäure, Pulver enthaltend 77 bis 87 Gew.-% Aktivsubstanz (INCI-Bezeichnung: Chitosan PCA) (Amerchol Corp.) ² Silikonemulsion, ca. 27 Gew.-% Silikonelastomeraktivsubstanz (INCI-Bezeichnung Silicone Quaternium-16/Glycidoxy Dimethicone Crosspolymer, Trideceth-12) (Dow Corning) | |

Bei Anwendung der Zusammensetzung auf menschlichem Haar ließ sich eine hervorragende, langanhaltende Formstabilisierung erzielen. Das Haar wirkte voll und voluminös.

## Patentansprüche

1. Haarbehandlungsmittel, enthaltend - bezogen auf sein Gewicht -
a) 0,05 bis 5 Gew.-% mindestens eines Chitosans und/oder Chitosanderivates und
b) 0,05 bis 5 Gew.-% mindestens eines elastomeren Silans oder Siloxans mit quaternären Ammoniumgruppen im Molekül in Form einer ÖI-in-Wasser (O/W-) oder Wasser-in-ÖI (W/O-) Emulsion oder Microemulsion, bei der das Silan oder Siloxan die Ölphase bildet, wobei das bzw. die elastomeren Silan(e) oder Siloxan(e) mit quaternären Ammoniumgruppen im Molekül erhalten wurden durch Reaktion von
(i) einer organischen quaternären Ammoniumverbindung mit Epoxidgruppen oder Halohydringruppen, wobei die organische quaternäre Ammoniumverbindung mit Epoxidgruppen ausgewählt ist aus Glycidyl-trimethylammoniumchlorid und/oder Glycidyl-trimethylammoniumbromid und wobei die die organische quaternäre Ammoniumverbindung mit Halohydringruppen ausgewählt ist aus
- (3-Chlor-2-hydroxypropyl)trimethylammoniumchlorid,
- (3-Chlor-2-hydroxypropyl)dimethyldodecylammoniumchlorid,
- (3-Chlor-2-hydroxypropyl)dimethyloctadecylammoniumchlorid,
- (3-Chlor-2-hydroxypropyl)trimethylammoniumbromid,
- (3-Chlor-2-hydroxypropyl)dimethyldodecylammoniumbromid,
- (3-Chlor-2-hydroxypropyl)-dimethyloctadecylammoniumbromid mit
(ii) einem Silan oder Siloxan mit Aminogruppen, in Gegenwart von
(iii) einem Verzweigungs-Agens, wobei das Verzweigungs-Agens ausgewählt ist aus Butandioldiglycidylether und/oder Polypropylenglycoldiglycidylether, und
(iv) einem Tensid, wobei das Tensid ausgewählt ist aus ethoxylierten Alkoholen der Formel
CₙH₂ₙ₊₁(OCH₂CH₂)ₓOH
in der n für Zahlen zwischen 8 und 20, und x für Zahlen von 8 bis 18 stehen, dispergiert in
(v) einer wäßrigen polaren Phase,
wobei das Mittel
0,1 bis 4,5 Gew.-% eines Neutralisationsproduktes von Chitosan mit Pyrrolidoncarbonsäure enthält.

2. Haarbehandlungsmittel nach Anspruch 1, **dadurch gekennzeichnet, daß** es - bezogen auf sein Gewicht - 0,25 bis 4 Gew.-%, besonders bevorzugt 0,5 bis 3,5 Gew.-%, weiter bevorzugt 0,75 bis 3 Gew.-% und insbesondere 1 bis 2 Gew.-% eines Neutralisationsproduktes von Chitosan mit Pyrrolidoncarbonsäure enthält.

3. Haarbehandlungsmittel nach einem der Ansprüche 1 oder 2, **dadurch gekennzeichnet, daß** das Tensid ausgewählt ist aus ethoxylierten Alkoholen der Formel
CₙH₂ₙ₊₁(OCH₂CH₂)ₓOH
in der n für 13 und x für 12 stehen.

4. Haarbehandlungsmittel nach einem der Ansprüche 1 bis 3, **dadurch gekennzeichnet, daß** die Emulsion oder Mikroemulsion bezogen auf ihr Gewicht einen Silikongehalt von 15 bis 40 Gew.-%, vorzugsweise von 20 bis 35 Gew.-% und insbesondere von 25 bis 30 Gew.-% aufweist.

5. Haarbehandlungsmittel nach einem der Ansprüche 1 bis 4, **dadurch gekennzeichnet, daß** es - bezogen auf sein Gewicht - 0,5 bis 70 Gew.-%, vorzugsweise 1 bis 60 Gew.-% und insbesondere 5 bis 25 Gew.-% anionische(s) und/oder nichtionische(s) und/oder kationische(s) und/oder amphotere(s) Tensid(e), enthält.

6. Haarbehandlungsmittel nach einem der Ansprüche 1 bis 5, **dadurch gekennzeichnet, daß** es - bezogen auf sein Gewicht 0,05 bis 7,5 Gew.-%, kationische(s) Polymer(e), enthält, wobei bevorzugte kationische(s) Polymer(e) ausgewählt ist/sind aus
- Poly(methacryloyloxyethyltrimethylammoniumchlorid) (INCI: Polyquaternium-37) und/oder;
- quaternisierten Cellulose-Derivaten (INCI: Polyquaternium 10) und/oder
- kationischen Alkylpolyglycosiden und/oder
- kationisertem Honig und/oder
- kationischen Guar-Derivaten und/oder
- polymeren Dimethyldiallylammoniumsalzen und deren Copolymeren mit Estern und Amiden von Acrylsäure und Methacrylsäure und/oder
- Copolymeren des Vinylpyrrolidons mit quaternierten Derivaten des Dialkylaminoalkylacrylats und -methacrylats und/oder
- Vinylpyrrolidon-Vinylimidazoliummethochlorid-Copolymeren und/oder
- quaterniertem Polyvinylalkohol und/oder
- Polyquaternium 2 und/oder
- Polyquaternium-7 und/oder
- Polyquaternium 17 und/oder
- Polyquaternium 18 und/oder
- Polyquaternium 24 und/oder
- Polyquaternium 27.

## Claims

1. A hair treatment agent containing, based on its weight,
a) from 0.05 to 5 wt.% of at least one chitosan and/or chitosan derivative and
b) from 0.05 to 5 wt.% of at least one elastomeric silane or siloxane having quaternary ammonium groups in the molecule in the form of an oil-in-water (O/W) emulsion or a water-in-oil (W/O) emulsion or microemulsion, in which the silane or siloxane forms the oil phase, wherein the elastomeric silane(s) or siloxane(s) having quaternary ammonium groups in the molecule were obtained by reacting
(i) an organic quaternary ammonium compound having epoxide groups or halohydrin groups, wherein the organic quaternary ammonium compound having epoxide groups is selected from glycidyl trimethylammonium chloride and/or glycidyl trimethylammonium bromide, and wherein the organic quaternary ammonium compound having halohydrin groups is selected from
- (3-chloro-2-hydroxypropyl)trimethylammonium chloride,
- (3-chloro-2-hydroxypropyl)dimethyldodecylammonium chloride,
- (3-chloro-2-hydroxypropyl)dimethyloctadecylammonium chloride,
- (3-chloro-2-hydroxypropyl)trimethylammonium bromide,
- (3-chloro-2-hydroxypropyl)dimethyldodecylammonium bromide,
- (3-chloro-2-hydroxypropyl)-dimethyloctadecylammonium bromide with
(ii) a silane or siloxane having amino groups, in the presence of
(iii) a branching agent, wherein the branching agent is selected from butanediol diglycidyl ether and/or polypropylene glycol diglycidyl ether, and
(iv) a surfactant, wherein the surfactant is selected from ethoxylated alcohols of formula
CₙH₂ₙ₊₁(OCH₂CH₂)ₓOH
in which n represents numbers of between 8 and 20 and x represents numbers of from 8 to 18, dispersed in
(v) an aqueous polar phase,
wherein the agent contains
from 0.1 to 4.5 wt.% of a neutralization product of chitosan and pyrrolidone carboxylic acid.

2. The hair treatment agent according to claim 1, **characterized in that** it contains, based on its weight, from 0.25 to 4 wt.%, particularly preferably from 0.5 to 3.5 wt.%, more preferably from 0.75 to 3 wt.% and in particular from 1 to 2 wt.% of a neutralization product of chitosan and pyrrolidone carboxylic acid.

3. The hair treatment agent according to claim 1 or 2, **characterized in that** the surfactant is selected from exthoxylated alcohols of formula
CₙH₂ₙ₊₁(OCH₂CH₂)ₓOH
in which the n represents 13 and the x represents 12.

4. The hair treatment agent according to one of claims 1 to 3, **characterized in that**, based on its weight, the emulsion or microemulsion has a silicone content of from 15 to 40 wt.%, preferably of from 20 to 35 wt.% and in particular of from 25 to 30 wt.%.

5. The hair treatment agent according to one of claims 1 to 4, **characterized in that** it contains, based on its weight, from 0.5 to 70 wt.%, preferably from 1 to 60 wt.% and in particular from 5 to 25 wt.% of anionic and/or nonionic and/or cationic and/or amphoteric surfactant(s).

6. The hair treatment agent according to one of claims 1 to 5, **characterized in that** it contains, based on its weight, from 0.05 to 7.5 wt.% of cationic polymer(s), preferred cationic polymer(s) being selected from
- poly(methacryloyloxyethyl trimethylammonium chloride) (INCI: polyquaternium-37) and/or;
- quaternized cellulose derivatives (INCI: polyquaternium-10) and/or
- cationic alkyl polyglycosides and/or
- catonized honey and/or
- cationic guar derivatives and/or
- polymeric dimethyldiallylammonium salts and copolymers thereof with esters and amides of acrylic acid and methacrylic acid and/or
- copolymers of vinylpyrrolidone with quaternized derivatives of dialkylamino alkylacrylate and methacrylate and/or
- vinylpyrrolidone-vinylimidazolium methochloride copolymers and/or
- quaternized polyvinyl alcohol and/or
- polyquaternium-2 and/or
- polyquaternium-7 and/or
- polyquaternium-17 and/or
- polyquaternium-18 and/or
- polyquaternium-24 and/or
- polyquaternium-27.

## Revendications

1. Agent de traitement capillaire contenant, sur la base de son poids,
a) de 0,05 à 5% en poids d'au moins une chitosane et/ou un dérivé de la chitosane et
b) 0,05 à 5% en poids d'au moins un silane ou siloxane élastomère comportant des groupes ammonium quaternaire dans la molécule sous la forme d'une émulsion ou microémulsion huile-dans-eau (H/E) ou eau-dans-huile (E/H) dans laquelle le silane ou le siloxane forme la phase huileuse,
le ou les silanes ou siloxanes élastomères pourvus de groupes ammonium quaternaire dans la molécule ayant été obtenus par réaction
(i) d'un composé d'ammonium quaternaire organique ayant des groupes époxyde ou des groupes halohydrine, le composé d'ammonium quaternaire organique pourvu de groupes époxy étant choisi parmi le chlorure de glycidyltriméthylammonium et/ou le bromure de glycidyltriméthylammonium et le composé d'ammonium quaternaire organique pourvu de groupes halohydrine étant choisi parmi
- le chlorure de (3-chloro-2-hydroxypropyl)triméthylammonium,
- le chlorure de (3-chloro-2-hydroxypropyl)diméthyldodécylammonium,
- le chlorure de (3-chloro-2-hydroxypropyl)diméthyloctadécylammonium,
- le bromure de (3-chloro-2-hydroxypropyl)triméthylammonium,
- le bromure de (3-chloro-2-hydroxypropyl)diméthyldodécylammonium,
- le bromure de (3-chloro-2-hydroxypropyl)diméthyloctadécylammonium avec
(ii) un silane ou un siloxane pourvu de groupes amino, en présence
(iii) d'un agent de ramification, l'agent de ramification étant choisi parmi l'éther diglycidylique de butandiolet/ou l'éther diglycidylique de polypropylène glycol ;
(iv) un tensioactif, le tensioactif étant choisi parmi les alcools éthoxylés de la formule
CₙH₂ₙ₊₁(OCH₂CH₂)ₓOH
dans laquelle n représente des nombres entre 8 et 20, et x représente des nombres de 8 à 18, dispersé dans
(v) une phase polaire aqueuse,
l'agent contenant
0,1 à 4,5% en poids d'un produit de neutralisation du chitosane avec de l'acide pyrrolidonecarboxylique.

2. Agent de traitement de cheveux selon la revendication 1, **caractérisé en ce qu'**il contient, sur la base de son poids, de 0,25 à 4% en poids, de manière particulièrement préférée de 0,5 à 3,5% en poids, de préférence 0,75 à 3% en poids et en particulier de 1 à 2% en poids d'un produit de neutralisation du chitosane avec l'acide pyrrolidonecarboxylique.

3. Agent de traitement capillaire selon la revendication 1 ou 2, **caractérisé en ce que** le tensioactif est choisi parmi les alcools éthoxylés de la formule
CₙH₂ₙ₊₁(OCH₂CH₂)ₓOH
dans laquelle n est 13 et x est 12.

4. Agent de traitement capillaire selon l'une des revendications 1 à 3, **caractérisé en ce que** l'émulsion ou la microémulsion a, sur la base de son poids, une teneur en silicone de 15 à 40% en poids, de préférence de 20 à 35% en poids et en particulier de 25 à 30% en poids.

5. Agent de traitement capillaire selon l'une des revendications 1 à 4, **caractérisé en ce qu'**il contient, sur la base de son poids, de 0,5 à 70% en poids, de préférence de 1 à 60% en poids et en particulier de 5 à 25% en poids d'un ou plusieurs tensioactifs anioniques et/ou non ioniques et/ou cationiques et/ou amphotères.

6. Agent de traitement capillaire selon l'une des revendications 1 à 5, **caractérisé en ce qu'**il contient, sur la base de son poids, de 0,05 à 7,5% en poids, d'un ou plusieurs polymères cationiques, le ou les polymères cationiques préférés étant sélectionnés parmi
- le polychlorure de méthacryloyloxyéthyltriméthylammonium (INCI : Polyquaternium-37) et/ou,
- les dérivés quaternisés de la cellulose (INCI : Polyquaternium 10) et/ou
- les polyglycosides d'alkyle cationique et/ou
- le miel cationisé et/ou
- les dérivés de guar cationiques et/ou
- les sels polymères de diméthyldiallylammonium et leurs copolymères avec des esters et des amides d'acide acrylique et d'acide méthacrylique et/ou
- les copolymères de vinylpyrrolidone avec des dérivés quaternisés de l'acrylate et du méthacrylate de dialkylaminoalkyle et/ou
- les copolymères vinylpyrrolidone-méthoxychlorure de vinylimidazolium et/ou
- l'alcool polyvinylique quaternisé et/ou
- le Polyquaternium 2 et/ou
- le Polyquaternium-7 et/ou
- le Polyquaternium 17 et/ou
- le Polyquaternium 18 et/ou
- le Polyquaternium 24 et/ou
- le Polyquaternium 27.
